# EUROPÄISCHE PATENTANMELDUNG

(11) **EP 2 602 001 A1**
(43) Veröffentlichungstag der Anmeldung: **12.06.2013**
(21) Anmeldenummer: 12190719.0
(22) Anmeldetag: 31.10.2012
(51) Int. Cl.: A61N 1/05, A61N 1/378

(54) **Medizintechnisches Implantat und medizintechnische Anordnung**

(30) Priorität: 08.12.2011 US 201161568176 P
(71) Anmelder: BIOTRONIK SE & Co. KG, 12359 Berlin (DE)
(72) Erfinder: Biela, Sarah, 12053 Berlin (DE); Skerl, Olaf, 18209 Bad Doberan (DE)
(74) Vertreter: Lindner-Vogt, Karin L.

(57) **Zusammenfassung**

Medizintechnisches Implantat, mit einem bei elektrischer und/oder magnetischer Ansteuerung das Implantat in mechanische Schwingungen versetzenden Schwinger-Element.

## Beschreibung

Die Erfindung betrifft ein medizintechnisches Implantat sowie eine ein solches Implantat umfassende medizintechnische Anordnung. Neben implantierbaren Elektrodenleitungen (im Folgenden auch kurz "Elektrodenleitungen" genannt) oder Sensorleitungen, wie sie etwa bei Herzschrittmachern oder implantierbaren Kardiowertern eingesetzt werden, betrifft der Begriff "Implantat" im Sinne dieser Patentanmeldung auch sogenannte "Leadless Pacers" (zuleitungsfreie Herzschrittmacher), wie sie etwa in der EP 1 714 670 B1 beschrieben sind, oder "Leadless Sensors" (zuleitungsfreie Sensoren), welche Sensoren mit einer Sende-/Empfangseinheit sind und die in das Gefäßsystem eines Lebewesens oder subkutan implantiert werden können.

Die Explantation von eingewachsenen Elektrodenleitungen stellt einen Arzt vor große Schwierigkeiten. Bis jetzt wird eine Elektrodenleitung bei Explantation "herausgeschnitten", beispielsweise durch Laserschneiden oder mit herkömmlichen Messern. Gleichzeitig muss an der Elektrodenleitung eine Zugkraft aufgebracht werden, welche aber nicht zum Reißen der Leitung führen darf. Es wurden spezielle Systeme zur Lösung dieser schwierigen Aufgabe entwickelt, so etwa ein Elektroden-Extraktions-System der Firma VascoMed GmbH.

Seit langem bekannt sind auch verschiedene Beschichtungen (etwa auf Polymerbasis), die ein Einwachsen von Elektrodenleitungen oder anderen medizintechnischen Implantaten im Körper erschweren sollen, oder auch Beschichtungen, welche Wirkstoffe abgeben (Drug-Eluting-Beschichtungen), die ein Einwachsen über diese Wirkstoffabgabe verhindern sollen.

Eine Beschichtung eines Implantats wird zum Beispiel erreicht, indem ein hydrophiles Interface zwischen die Implantatoberfläche und der Körperflüssigkeit eingebettet wird. Dadurch werden entzündliche Reaktionen des umliegenden Gewebes sowie Einwachsungen minimiert. Mehrere natürliche, synthetische und semisynthetische Materialien werden derzeit genutzt für Implantatbeschichtungen. Natürlich vorkommende Materialien beinhalten Alginat, Chitosan, Collagen, Dextran und Hyaluronan, synthetische Polymermaterialien als Beschichtungsmaterialien sind z.B. Poly(lactic-acid) und Poly(lactic co-glycolic acid) (PLGA), 2-Hydroxyethyl-Methacrylate, Poly(ethyleneglycol) (PEG), und Poly(vinylalcohol) (PVA). Nicht alle dieser Materialien zeigen dauerhaft die gewünschten Eigenschaften. Manche liefern nicht die ausreichende mechanische Belastbarkeit, chemische Stabilität oder Biokompatibilität für alle Anwendungen, so dass ein Einwachsen nicht vollständig verhindert werden kann.

Die Fremdkörperreaktion auf ein Implantat kann minimiert oder sogar kontrolliert werden durch den Einsatz von steroiden und nichtsteroiden entzündungshemmenden Arzneimitteln. Bisher wurden beispielsweise Glukocortikoide verwendet.

Auf lange Sicht wachsen die meisten Elektrodenleitungen an den Stellen ein, an denen sie wandständig im Blutgefäß liegen, und lassen sich dadurch nicht mehr oder nur sehr schwer explantieren. Viele der oben genannten Materialien eignen sich nur für einen begrenzten Zeitraum und können dauerhaft die Fremdkörperreaktion und somit ein Einwachsen von Implantaten, insbesondere Elektrodenleitungen, Sensoren (leitungsgebunden oder zuleitungsfrei) oder Leadless Pacern nicht verhindern. Bisher sind keine Beschichtungen, weder reine Polymere, noch Drug-Eluting-Polymere bekannt, die das Problem über längere Zeiträume als mehrere Monate lösen.

Beim Explantieren, besonders von Elektrodenleitungen in Herznähe besteht auch bei Einsatz speziell entwickelter Systeme und bei großer Erfahrung des Operateurs ein hohes Risiko, wichtige Blutgefäße oder das Herz zu verletzen, insbesondere dann, wenn die Elektrodenleitungen eingewachsen sind. Deswegen bleiben sie oft im Körper, wenn sie ersetzt werden müssen.

Die Risiken, die eine verbleibende Elektrodenleitung im Körper mit sich bringt, sind zwar schwer abzuschätzen, werden aber momentan in Kauf genommen, um die erheblich höheren Risiken einer Explantation nicht eingehen zu müssen.

Der Erfindung liegt die Aufgabe zugrunde, ein im Hinblick auf eine mögliche Explantation praxistauglicheres medizintechnisches Implantat anzugeben.

Diese Aufgabe wird durch ein Implantat mit den Merkmalen des Anspruchs 1 gelöst. Zweckmäßige Fortbildungen des Erfindungsgedankens sind Gegenstand der abhängigen Ansprüche. Es wird des Weiteren eine medizintechnische Anordnung mit den Merkmalen des Anspruchs 14 bereitgestellt.

Die Erfindung schließt die Überlegung ein, von der bisher üblichen Anwendung hoher Zugkräfte zur Explantation von medizintechnischen Implantaten gemäß der eingangs genannten Definition zur Herauslösung aus der Gewebsumgebung, in die sie eingewachsen sind, abzugehen. Sie schließt weiter die Überlegung ein, das Implantat zum Lösen aus der Gewebsumgebung stattdessen in Schwingungen zu versetzen, also gewissermaßen zu "rütteln". Schließlich gehört zur Erfindung der Gedanke, die mechanischen Schwingungen nicht außerhalb des Körpers zu erzeugen und durch ein mechanisches Übertragungselement auf das Implantat zu übertragen, sondern sie durch geeignete Energieumwandlung direkt im Implantat zu erzeugen. Zu diesem Zweck wird im Implantat ein Schwinger-Element vorgesehen, welches bei elektrischer und/oder magnetischer Ansteuerung das Implantat in mechanische Schwingungen versetzt und dadurch ein Lösen aus der Gewebsumgebung oder zumindest ein Lockern in dieser bewirkt. Es sei darauf hingewiesen, dass dieses Lösen oder Lockern nicht notwendigerweise nur zum Zweck und Zeitpunkt einer Explantation stattfinden muss, sondern ggfs. auch präventiv zur Verhinderung eines zu festen Einwachsens, etwa in größeren Abständen während der Funktionsdauer eines Langzeit-Implantats, erfolgen könnte.

Ein Schwinger-Element in der Spitze einer Elektroden- oder Sensorleitung oder in einem Leadless Pacer/Sensor oder einem ähnlichen Implantat wird die Entwicklung künftiger Implantatbeschichtungen enorm erleichtern, da es dadurch weniger notwendig ist, körpereigene Zellen und Einwachsungen fernzuhalten. Ein geringer Grad an Einwachsung kann toleriert werden, denn die Erfindung ermöglicht eine Explantation trotz Einwachsung in Körpergewebe.

Zudem werden Drug-Eluting-Beschichtungen, die dem Körper schaden können, vermieden. Man kann gezielt zum Zeitpunkt der Explantation steuern, wann sich das Implantat vom Gewebe lösen soll.

Ein weiterer Vorteil bietet sich, da die körpereigenen Einwachsungen die Lage des Implantats zusätzlich stabilisieren. Dies ist von besonderer Bedeutung für leitungsgebundene oder Leadless Sensoren, die sich in der Pulmonalarterie oder auch der Vena Cava vor dem Herzen befinden. Ein Verrutschen der Sensoren könnte zu schwerwiegenden Komplikationen führen.

In einer Ausführung der Erfindung umfasst das Implantat ein induktiv mit einem magnetischen Wechselfeld koppelndes Schwinger-Element. In einer aus derzeitiger Sicht allerdings bevorzugten Ausführung handelt es sich um ein elektrisch ansteuerbares Schwinger-Element wie beispielsweise ein piezokeramisches Schwing-Element.

In einer weiteren Ausführung ist das Schwinger-Element als in das Implantat eingefügter separater Schwingkörper ausgebildet. In einer anderen Ausführung ist das Schwinger-Element im Wandungsbereich des Implantats angeordnet, insbesondere großflächig in die Wandung eingebettet. Wird die letztgenannte Ausführung mit der Realisierung als elektrisch ansteuerbares Piezo-Element verbunden, ergibt sich als vorteilhafte Ausgestaltung, dass das Schwinger-Element eine piezokeramische Folie oder piezoelektrische Polymerfolie aufweist.

Die erwähnten Piezofolien sind wenige µm dick und vergrößern den Durchmesser eines Implantats unwesentlich. Sie lassen sich beispielsweise schlauchförmig herstellen, was bei der Produktion einige Vorteile ergibt. Auch sollte vorgesehen werden, dass sich die Piezofolien möglichst entlang der kompletten Längsachse des Implantats erstreckt, denn es kann nicht genau gesagt werden, wo ein Implantat in das Gewebe einwächst.

In einer weiteren Ausführung der Erfindung hat das vorgeschlagene Implantat einen elektrischen Anschluss zur Kontaktierung des Schwinger-Elements mittels einer temporären, als Explantationshilfsmittel dienenden Ansteuerleitung. In einer anderen Ausführung ist eine integrierte Ansteuerleitung zur Verbindung mit einer internen oder in einem weiteren Implantat angeordneten oder extrakorporalen Ansteuereinrichtung vorgesehen.

In einer noch anderen Ausführung ist das Schwinger-Element derart ausgebildet oder es sind zusätzliche Energieversorgungsmittel derart vorgesehen, dass eine drahtlose Ansteuerung über ein extrakorporal erzeugtes elektromagnetisches Wechselfeld (erzeugt etwa durch eine in der Nähe des Implantats an den Körper gehaltenen Erregerspule) erfolgen kann. Ein Vorteil der drahtlosen Energieversorgung des Schwinger-Elements wäre, dass keine zusätzliche Technik im Hauptimplantat und keine zusätzliche Elektrodenleitung nötig sind.

In einer aus derzeitiger Sicht besonders wichtigen Anwendung der Erfindung ist das Implantat ausgebildet als implantierbare Elektroden- oder Sensorleitung. In einer weiteren perspektivisch interessanten Ausführung handelt es sich um ein zuleitungsfreies Herzstimulations- oder Kardioversionsgerät oder um einen leitungsgebundenen oder zuleitungsfreien Sensor für intrakorporale, physikalische, physiologische oder biochemische Messgrößen, welche an sich beispielsweise mit einer zylindrischen Grundform bekannt ist. In beiden Anwendungsformen kann vorteilhaft eine piezokeramische Folie oder piezoelektrische Polymerfolie, insbesondere in Hülsen- oder Ringsegmentform, in oder auf der Wandung eines distalen Abschnitts angeordnet sein.

Die vorgeschlagene medizintechnische Anordnung, die ein Implantat der oben erläuterten Art umfasst, hat im Übrigen eine Ansteuereinrichtung zur elektrischen und/oder magnetischen Ansteuerung des Schwinger-Elements und Kopplungsmittel zur Energieeinkopplung in dieses. Es kann sich hierbei um eine insgesamt implantierbare Anordnung (etwa aus einer Schrittmacher-Elektrodenleitung und einem speziell ausgestatteten Herzschrittmacher) handeln. Klinisch interessanter ist aus derzeitiger Sicht jedoch eine Anordnung, bei der die Ansteuereinrichtung als extrakorporales Explantationsunterstützungsgerät ausgebildet ist.

Vorteile und Zweckmäßigkeiten der Erfindung ergeben sich im Übrigen aus der nachfolgenden skizzenartigen Beschreibung von Ausführungsbeispielen anhand der Figuren. Von diesen zeigen:
- Fig. 1A: eine Prinzipskizze einer ersten Ausführung des erfindungsgemäßen Implantats am Beispiel eines Abschnittes des Elektrodenkörpers einer Elektrodenleitung,
- Fig. 1B: eine Prinzipskizze einer weiteren Ausführung des erfindungsgemäßen Implantats am Beispiel einer Elektrodenleitung,
- Fig. 2: eine Prinzipskizze einer weiteren Ausführung des erfindungsgemäßen Implantats am Beispiel einer Elektrodenleitung,
- Fig. 3: eine Prinzipskizze einer weiteren Ausführung des erfindungsgemäßen Implantats am Beispiel einer Elektrodenleitung,
- Fig. 4: eine Prinzipskizze einer weiteren Ausführung des erfindungsgemäßen Implantats am Beispiel eines zuleitungsfreien Schrittmachers,
- Fig. 5: eine schematische Darstellung einer ersten Ausführungsform der erfindungsgemäßen medizintechnischen Anordnung und
- Fig. 6: eine schematische Darstellung einer weiteren Ausführung der vorgeschlagenen medizintechnischen Anordnung.

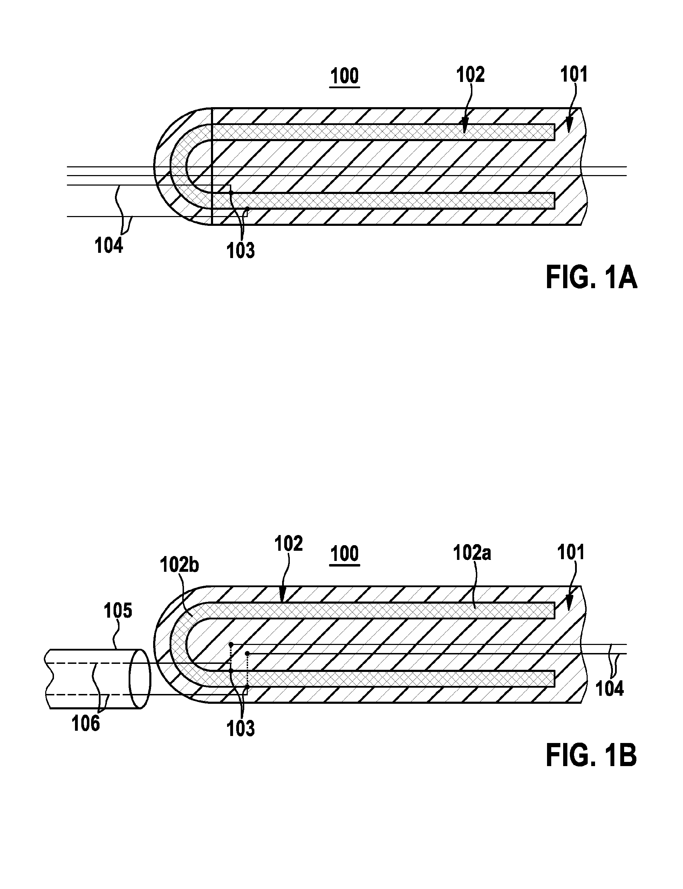

Fig. 1A und 1B zeigen schematisch nur die im Zusammenhang mit der vorliegenden Erfindung wesentlichen Teile des distalen Endes einer Elektrodenleitung 100, die im Übrigen - in an sich bekannter Weise - einen oder mehrere Elektrodenpole zur Stimulation von reizbarem Körpergewebe und/oder zum Abfühlen von Gewebspotentialen und ggfs. auch einen oder mehrere Sensoren zur Erfassung weiterer physiologischer Größen im Körper eines Patienten umfasst. Jene Teile und deren Zu- und Ableitungen sind nicht dargestellt und werden hier nicht weiter beschrieben, da sie dem Fachmann vertraut sind. Des Weiteren lassen sich die konkret anhand einer Elektrodenleitung beschriebenen Merkmale auch in den anderen unter dem Begriff "Implantat" zusammengefassten Vorrichtungen einsetzen.

Fig. 1A zeigt einen Längsschnitt eines Elektrodenkörperabschnittes einer Elektrodenleitung 100. In einen Kunststoffmantel 101 der Elektrodenleitung 100 ist eine piezokeramische Folie 102 eingebettet, die in der hier gezeigten Ausführung eine langgestreckte Hülse umfasst. Die piezokeramische Folie erstreckt sich über zumindest einen Abschnitt des Elektrodenkörpers, der sich zwischen dem distalen, zur Behandlungsstelle weisenden Ende und einem proximalen, in Richtung des implantierten elektromedizinischen Gerätes erstreckt. Des Weiteren verlaufen im Elektrodenkörper Zuleitungen 107 zur elektrischen Verbindung der therapeutischen oder sensorischen Elektroden am distalen Ende der Elektrodenleitung. Über Anschlusskontakte 103 an der inneren und äußeren Folienoberfläche ist die piezokeramische Folie 102 mit elektrischen Zuleitungen 104 im Inneren des Elektrodenkörpers verbunden, die ihrerseits mit einem ebenfalls implantierten elektromedizinischen Gerät oder - speziell im Falle einer Explantation - mit einem externen Gerät verbunden sind. Wie in der folgenden Ausführung gemäß Fig. 1B dargestellt, kann die piezokeramische Folie auch über ein im Explantationsfall herangeführtes separates Kabel 105 mit darin vorgesehenen Zuleitungen 106 extern kontaktierbar sein.

Wird die Piezofolie 102 mit einer Wechselspannung geeigneter Frequenz und Spannung beaufschlagt, strahlt sie akustische Wellen ab. Die abgestrahlten akustischen Wellen bewirken die Trennung oder Lockerung des Bewuchses von der Oberfläche der Elektrode. Die Frequenz der aufgeprägten Wechselspannung kann im Bereich von 20 kHz bis zu 20 MHz liegen, vorzugsweise wird eine Frequenz zwischen 50 kHz und 100 kHz verwendet. Die Wechselspannung kann als kontinuierliche Wechselspannung beliebiger Kurvenform oder in Form von Bursts oder Impulsen aufgeprägt werden.

In Abwandlung der dargestellten Ausführung kann das Schwinger-Element mit ringförmigen Segmenten, die elektrisch miteinander verbunden sind, ausgeführt sein, und es kann aus einem piezoelektrischen Material, z.B. aus Blei-Zirkonat-Titant (PZT), Bariumtitanat oder Lithiumniobat, bestehen. Durch die ringförmigen Segmente wird die Flexibilität der Elektrode in diesem Bereich erhöht. Alternativ zur piezokeramischen Folie kann auch eine Folie aus einem piezoelektrischen Polymer (z.B. PVDF; Polyvinylidenfluorid) verwendet werden.

Fig. 1B zeigt eine weitere Ausführung einer Elektrodenleitung 100. Dargestellt ist das distale Ende Elektrodenleitung 100. In einen Kunststoffmantel 101 der Elektrodenleitung 100 ist eine piezokeramische Folie 102 eingebettet, die in der hier gezeigten Ausführung eine langgestreckte Hülse 102a und eine halbkugelförmige Kappe 102b im Bereich der (ebenfalls halbkugelförmigen) distalen Elektrodenspitze umfasst. Über Anschlusskontakte 103 an der inneren und äußeren Folienoberfläche ist die piezokeramische Folie 102 entweder (hier dargestellt über punktierte Linien) mit elektrischen Zuleitungen 105 im Inneren des Elektrodenkörpers verbunden, die ihrerseits mit einem ebenfalls implantierten elektromedizinischen Gerät oder - speziell im Falle einer Explantation - mit einem externen Gerät über einen am proximalen Ende der Elektrodenleitung 100 vorhandenen und nicht dargestellten Elektrodenstecker elektrisch verbunden sind, oder sie ist über ein im Explantationsfall herangeführtes separates Kabel 105 mit darin vorgesehenen Zuleitungen 106 extern kontaktierbar. Zur besseren Darstellung des Prinzips ist das Kabel 105 schematisch von distaler Seite an die piezokeramische Folie herangeführt. Selbstverständlich erschließt es sich dem Fachmann, dass dieses Kabel im Falle der Explantation in das Innere des Elektrodenkörpers von proximaler Richtung her eingeführt ist.

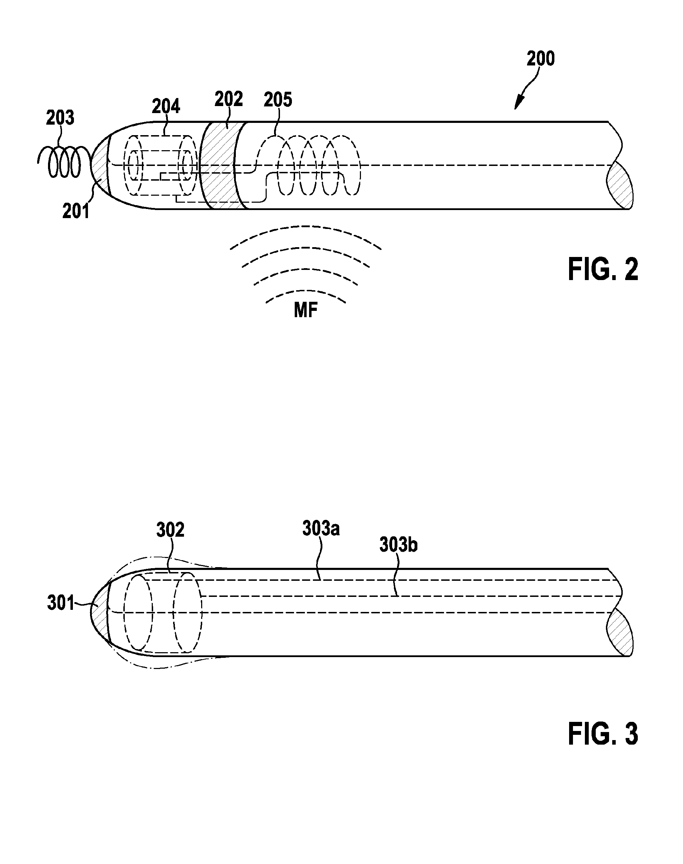

Fig. 2 zeigt skizzenartig als Abwandlung der in Fig. 1B dargestellten Ausführung das distale Ende einer Elektrodenleitung 200 mit einer Spitzenelektrode 201 und einer Ringelektrode 202, an deren distalem Ende eine Fixierungswendel 203 zur Verankerung in zu stimulierendem Körpergewebe, etwa im Trabekelwerk eines Herzens, vorgesehen ist. Bei der Elektrodenleitung 200 ist nahe dem distalen Ende als Schwinger-Element ein Piezokeramik-Schwingkörper 204 in Form eines Hohlzylinders eingebaut, dessen Innen- und Außenwandung mit den Enden einer Empfangsspule 205 verbunden sind.

Über diese Spule 205 wird die Energie zur Erzeugung der akustischen Welle drahtlos mittels magnetisch-induktiver Kopplung eingespeist. Bei der Explantation geschieht dies über eine geeignete (nicht dargestellte) Sendespule, die von außen an den Körper gehalten wird. Diese Lösung hat den Vorteil, dass die Elektrode keine zusätzlichen Anschlüsse benötigt und in dem IMD keine zusätzlichen speziellen Vorrichtungen zum Erzeugen und Einspeisen der Wechselspannung notwendig sind. Damit ist diese Elektrode vollständig kompatibel zu herkömmlichen Elektrodenanschlüssen und IMDs.

Fig. 3 zeigt als weitere Ausführung eine Elektrodenleitung 300, die als einzige Elektrode eine Spitzenelektrode 301 umfasst und deren Ende - was durch die gestrichelt gezeichnete Ausbauchung des distalen Endes symbolisiert wird - zur Vermeidung einer Penetration von Körperwandungen bei Wandkontakt elastisch kompressibel ausgeführt ist. Nahe dem distalen Ende ist zur Bestimmung einer Andruckkraft bei Wandkontakt der Elektrodenspitze ein kapazitiver Drucksensor 302 (etwa mit einem kompressiblen leitfähigen Schaum) vorgesehen. Dieser ist an seiner distalen und proximalen Stirnfläche jeweils über eine Elektrode und einer dort angeschlossenen Zuleitung 303a, 303b mit einem (nicht dargestellten) proximalen Anschlusskontakt der Elektrodenleitung verbunden.

Hiermit wird eine weitere Möglichkeit zur Erzeugung der benötigten akustischen Wellen genutzt. Kapazitive Drucksensoren können zur Erzeugung akustischer Wellen verwendet werden, indem an die druckmessende Kapazität eine Wechselspannung angelegt wird. Sie arbeiten dann (quasi invers) als CMUTs (Capacitive Micromachined Ultrasonic Transducers). Die Vorrichtung zur Erzeugung der Wechselspannung kann beispielsweise mit in den Vorrichtungen zur Bestimmung des Drucksignals aus der Kapazität des kapazitiven Drucksensors enthalten sein. Alternativ kann diese Wechselspannung ebenfalls wieder über eine geeignete Spule drahtlos magnetisch-induktiv eingekoppelt werden.

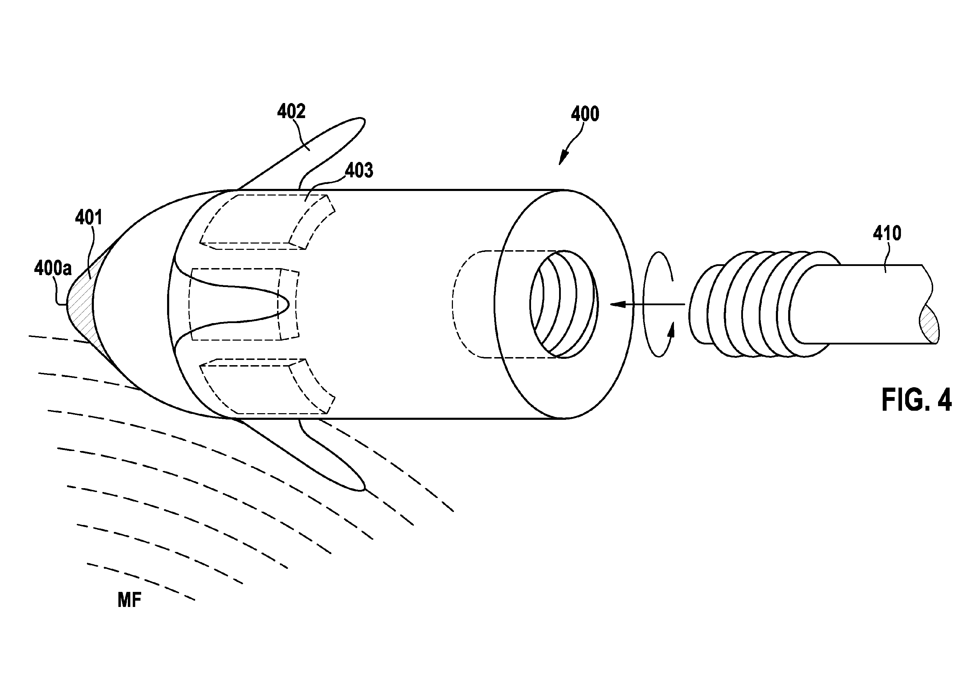

Fig. 4 zeigt als weitere Ausführung der Erfindung einen zuleitungsfreien Schrittmacher 400, dessen Grundgestalt zylindrisch ist und dessen eines Ende in eine abgerundete Spitze 400a ausläuft, an der eine Stimulationselektrode 401 angeordnet ist. Kunststoff-Finnen 402 nahe diesem Ende des Schrittmachers 400 sind zur Verankerung in verästelten Körpergeweben am Einsatzort des Schrittmachers vorgesehen. Neben den üblichen Komponenten eines derartigen Gerätes umfasst der Schrittmacher 400 einen Ring aus über ein externes magnetisches Wechselfeld MF induktiv zu Schwingungen anregbaren Schwingkörpern 403, die nahe dem Anbringungspunkt der Finnen 402 platziert sind. Sie regen speziell die Finnen 402 zu elastischen Schwingungen an, die deren Verankerung im verästelten Körpergewebe lockern, und schaffen so die Voraussetzungen für eine Explantation des Schrittmacher 400 mittels eines (hier symbolisch als Führungsdraht mit entständigem Außengewinde dargestellten) Explantationswerkzeugs 410. Das Explantationswerkzeug 410 gemäß dieser Ausführung kann auch als weitere Ausgestaltung der zu Fig. 1 und 2 genannten Kontaktmöglichkeit zu den genannten Schwingkörpern 403 dienen. In diesem Fall wird die elektrische Energie über eine galvanische Kontaktierung zwischen Explantationswerkzeug - insbesondere über elektrisch leitfähige Kontakte an der Oberfläche des Explantationswerkzeuges - und Schwingkörpern realisiert. Kontaktflächen im Inneren des zuleitungsfreien Schrittmachers 400 stellen diesen Kontakt sicher. Selbstverständlich ist diese Art von Einkopplung der Energie auch bei den anderen unter dem hier definierten Begriff "Implantat" möglich.

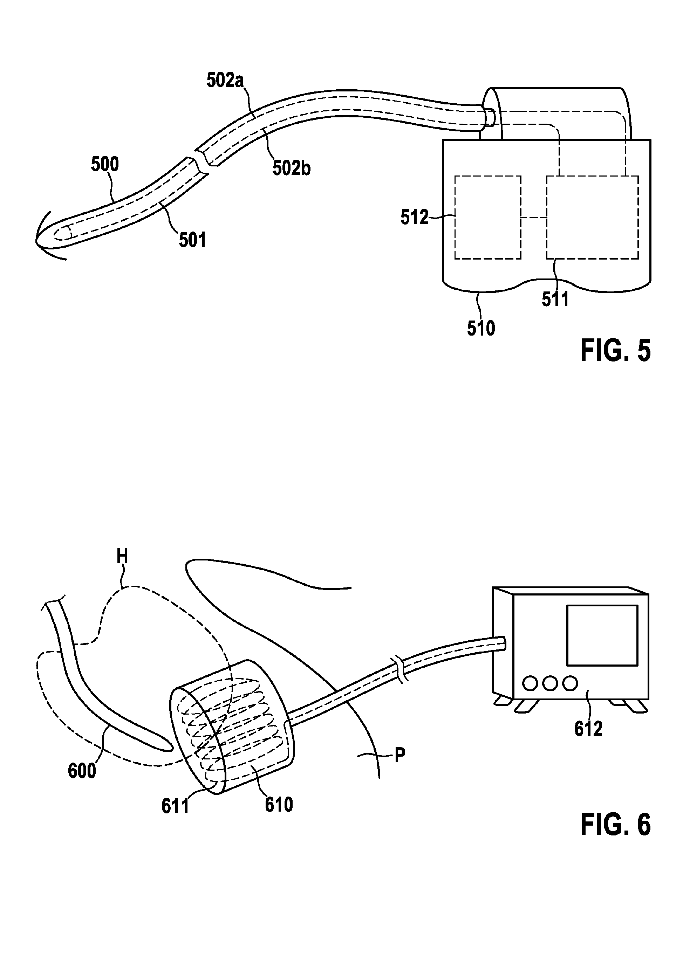

Fig. 5 zeigt schematisch als erstes Beispiel einer erfindungsgemäßen medizintechnischen Anordnung eine Elektrodenleitung 500 mit nahe ihrem distalen Ende eingebetteter Piezofolie 501 und zwei elektrischen Zuleitungen 502a, 502b zu jener, die mit einem Herzschrittmacher 510 derart verbunden ist, dass die Leitungen 502a, 502b im Schrittmacher mit einem Explantations-Schwingungserzeuger 511 verbunden sind. Dieser wird zur Vorbereitung einer Explantation der Leitung 500 aktiviert und über die Schrittmacherbatterie 512 für einen vorbestimmten Zeitraum mit Energie versorgt, um Schwingungen zum Lösen des Leitungsendes aus umgebendem Herzgewebe bereitzustellen.

Als alternative Ausführung zeigt Fig. 6 das distale Ende einer im Herzen H eines Patienten P verlegten Elektrodenleitung 600, die in Art der in Fig. 2 gezeigten Ausführung mit einer Piezokeramik in Verbindung mit einer Empfangsspule oder auch in Art des in Fig. 4 dargestellten "Leadless Pacers" mit einem induktiv angetriebenen Schwingkörper ausgestattet ist. Zur Vorbereitung einer Explantation dieser Elektrodenleitung wird von außen an den betreffenden Körperbereich ein Energieversorgungskopf 610 herangeführt, der eine Sendespule 611 enthält und mit einem externen Versorgungs- und Steuergerät 612 verbunden ist. Die Energiezufuhr aus dem Energieversorgungskopf 610 in das (nicht separat dargestellte) Schwinger-Element in der Elektrodenleitung 600 erfolgt auf die weiter oben beschriebene Weise mittels eines elektromagnetischen Wechselfeldes. Die Wechselspannung hierfür wird durch einen im Versorgungs- und Steuergerät 612 enthaltenen Generator für eine geeignete Zeitspanne bereitgestellt, die einerseits ausreichend zum Lockern der Elektrodenleitung und andererseits gesundheitlich unbedenklich für den Patienten ist.

Die Ausführung der Erfindung ist nicht auf die oben beschriebenen Beispiele und hervorgehobenen Aspekte beschränkt, sondern ebenso in einer Vielzahl von Abwandlungen möglich, die im Rahmen fachgemäßen Handelns liegen.

## Patentansprüche

1. Medizintechnisches Implantat, mit einem bei elektrischer und/oder magnetischer Ansteuerung das Implantat in mechanische Schwingungen versetzenden Schwinger-Element.

2. Implantat nach Anspruch 1, mit einem induktiv mit einem magnetischen Wechselfeld koppelnden Schwinger-Element.

3. Implantat nach Anspruch 1, mit einem elektrisch ansteuerbaren Schwinger-Element.

4. Implantat nach einem der vorangehenden Ansprüche, wobei das Schwinger-Element als in das Implantat eingefügter separater Schwingkörper ausgebildet ist.

5. Implantat nach Anspruch 1, wobei das Schwinger-Element im Wandungsbereich des Implantats angeordnet, insbesondere großflächig in die Wandung eingebettet ist.

6. Implantat nach Anspruch 3, wobei das Schwinger-Element eine piezokeramische Folie oder piezoelektrische Polymerfolie aufweist.

7. Implantat nach Anspruch 1, mit einem elektrischen Anschluss zur Kontaktierung des Schwinger-Elements mittels einer temporären, als Explantationshilfsmittel dienenden Ansteuerleitung.

8. Implantat nach Anspruch 1, mit integrierter Ansteuerleitung zur Verbindung mit einer internen oder in einem weiteren Implantat angeordneten oder extrakorporalen Ansteuereinrichtung.

9. Implantat nach Anspruch 1, wobei das Schwinger-Element ausgebildet ist oder zusätzliche Energieversorgungsmittel vorgesehen sind zur drahtlosen Ansteuerung über ein extrakorporal erzeugtes elektromagnetisches Wechselfeld.

10. Implantat nach Anspruch 9, wobei das Implantat eine in das Schwinger-Element eingefügte oder mit diesem verbundene Empfangsspuleneinrichtung aufweist.

11. Implantat nach Anspruch 1, ausgebildet als implantierbare Elektroden- oder Sensorleitung.

12. Implantat nach Anspruch 1, ausgebildet als zuleitungsfreies Herzstimulations- oder Kardioversionsgerät oder als zuleitungsfreier Sensor.

13. Implantat nach Anspruch 11, wobei eine piezokeramische Folie oder piezoelektrische Polymerfolie, insbesondere in Hülsen- oder Ringsegmentform, in oder auf der Wandung eines distalen Abschnitts angeordnet ist.

14. Medizintechnische Anordnung mit einem Implantat nach Anspruch 1 und einer Ansteuereinrichtung zur elektrischen und/oder magnetischen Ansteuerung des Schwinger-Elements und Kopplungsmitteln zur Energieeinkopplung in dieses.

15. Anordnung nach Anspruch 14, wobei die Ansteuereinrichtung als extrakorporales Explantationsunterstützungsgerät ausgebildet ist.
